# EUROPEAN PATENT APPLICATION

(11) **EP 3 388 528 A1**
(43) Date of publication of application: **17.10.2018**
(21) Application number: 17166655.5
(22) Date of filing: 13.04.2017
(51) Int. Cl.: C12P 41/00, C12P 7/02

(54) **PROCESS FOR PREPARATION OF A PRODUCT VIA A CHEMO-ENZYMATIC REACTION**

(71) Applicant: Technische Universität Hamburg-Harburg, 21073 Hamburg (DE); SU Holding, 10691 Stockholm (SE)
(72) Inventor: Liese, Andreas, 21073 Hamburg (DE); Kühn, Steffen, 21073 Hamburg (DE); Smirnova, Irina, 21075 Hamburg (DE); Christlieb, Marc-Andreas, 21073 Hamburg (DE); Bäckvall, Jan-Erling, 18278 Stocksund (SE); Yang, Bin, 18354 Täby (SE)
(74) Representative: Uexküll & Stolberg

(57) **Abstract**

The present invention relates to a process for the preparation of a product via a chemo-enzymatic reaction in a reactor comprising a first reaction zone comprising a chemo-catalyst, and second reaction zone comprising a biocatalyst, wherein the first reaction zone is operated at a first temperature T₁ to provide a first reaction mixture and the second reaction zone is operated at a second temperature T₂ which is lower than the first temperature T₁ to provide a second reaction mixture, wherein at least a part of the first reaction mixture is transferred to the second reactor zone and/or at least a part of the second reaction mixture is transferred to the first reactor zone, and
wherein the product is isolated from the first reaction mixture and /or the second reaction mixture.

## Description

### FIELD OF THE INVENTION

The present invention relates to a process for the preparation of a product via a chemo-enzymatic reaction. In particular, the present invention relates to a process for the preparation of a product via a chemo-enzymatic reaction in a one-pot reactor setup with spatial separated catalysts of different species.

### BACKGROUND OF THE INVENTION

The combination of chemocatalysis and biocatalysis in multistep organic synthetic processes is of high interest. As other types of synthetic processes, such chemo-enzymatic reactions employ catalysts to accelerate product formation. To obtain the desired product, it is possible to perform the reactions with the chemo-catalyst and an enzyme as the biocatalyst each being operated in different reactors or to use a mixture of a chemo-catalyst and a biocatalyst in one reactor.

The first alternative, i.e. use of chemo-catalyst and biocatalyst in different reactors typically requires use of different isolating or purification steps, which can often be complicated, time-consuming and/or expensive. The same problem applies to the three step reaction for the epoxidation of monoterpenes as described by Ranganathan et al (Journal of Molecular Catalysis B: Enzymatic 114 (2015) 72-76), where the three reaction steps are performed consecutively with at least one tedious work-up procedure between the reaction steps. Hence, in order to avoid this disadvantage chemo-enzymatic reactions are often carried out in accordance with the second alternative, i.e. by using the chemo-catalyst and the biocatalyst in the same reactor.

For example, a process for the preparation of a product via a chemo-enzymatic reaction is disclosed by Petrenz et al (Journal of Molecular Catalysis B: Enzymatic 114 (2015) 42-49). This document discloses the chemo-enzymatic dynamic kinetic resolution of rac-benzoin with Lipase TL and ZR-TUD-1 in one vessel at a temperature of 50 °C.

Moreover, Bäckvall et al (Current Opinion in Chemical Biology 2007, 11:226-232) give an overview on dynamic kinetic resolution reactions catalyzed by enzymes and metals. All discussed chemo-enzymatic reactions are run in one vessel at one temperature of up to 90 °C.

Furthermore, a horizontal reactive distillation apparatus is disclosed by Au-Yeung et al (Journal of American Institute of Chemical Engineers 2013, 59:2603-2620). Reduced overall temperatures of 27 °C are achieved by applying vacuum conditions to decrease the boiling points of the corresponding substrates and products.

However, the simultaneous use of a chemo-catalyst and a biocatalyst in the same reactor typically results in the problem that not both catalysts are operated at their optimum conditions. Biocatalysts are known to be temperature sensitive and typically degrade at high temperatures, while chemo-catalysts like metal catalysts usually reveal higher stability and higher activities (following Arrhenius equation) at increased temperatures. Thus, in order to avoid fast deactivation of the biocatalyst, rather low temperatures need to be applied in the single reaction vessel resulting in that the chemo-catalyst is not operated at its optimum reaction conditions or is even not operating at all. Consequently, there is still a demand for a process for chemo-enzymatic reactions avoiding the above mentioned drawbacks.

### SUMMARY OF THE INVENTION

Accordingly, the object of the present invention is to provide a process for the preparation of a product via a chemo-enzymatic reaction, which process allows that both the chemo-catalyst and the biocatalyst exhibit a high activity and stability and at the same time intermediate work-up and/or isolating steps are avoided or reduced.

To solve this problem, the present invention provides a process for the preparation of a product via a chemo-enzymatic reaction in a reactor comprising a first reaction zone comprising a chemo-catalyst, and second reaction zone comprising a biocatalyst, wherein the first reaction zone is operated at a first temperature T₁ to provide a first reaction mixture and the second reaction zone is operated at a second temperature T₂ which is lower than the first temperature T₁ to provide a second reaction mixture, wherein at least a part of the first reaction mixture is transferred to the second reactor zone and/or at least a part of the second reaction mixture is transferred to the first reactor zone, and wherein the product is isolated from the first reaction mixture and/or the second reaction mixture.

It has surprisingly been found that the spatial separation of chemo- and biocatalysts in different reaction zones of a one-pot reactor setup according to the invention enables both catalysts being operated at advantageous process conditions. Preferably, the chemo-catalyst can be operated at elevated temperatures increasing its activity, whereas deactivation of the biocatalyst operated at lower temperatures is prevented or at least substantially reduced. Furthermore, contrary to a multi-reactor setup, no cumbersome or time-consuming work-up steps are necessary between both catalytic reactions. In addition, the process in the one-pot reactor setup according to the invention optionally allows the recycling of components, which enables improved conversion by repeated contact with catalysts or interactive reactions, such as in a dynamic kinetic resolution, or other recirculation processes.

### BRIEF DESCRIPTION OF THE DRAWINGS

Fig. 1 is a schematic drawing of a one-pot reactor setup useful in a preferred embodiment of the process according to the invention.
Fig. 2 is a diagram showing the enantiomeric excess (ee) over the course of conversion for different temperatures for a chemo-enzymatic dynamic kinetic resolution reaction according to the Reference Example.
Fig. 3 is a diagram showing the temperature profiles of different reaction zones dependent on the reaction time for a chemo-enzymatic dynamic kinetic resolution reaction according to Example 1.
Fig. 4 is a diagram showing the concentration profiles of substrate materials (left), formed products (right) and solvent p-xylene (both) dependent on the reaction time for a spatially separated chemo-enzymatic dynamic kinetic resolution reaction according to Example 1.
Fig. 5 is a diagram showing the enantiomeric excess over the course of conversion for a spatially separated chemo-enzymatic dynamic kinetic resolution reaction according to Example 1.
Fig. 6 is a diagram showing the enantiomeric excess over the course of conversion for a spatially separated chemo-enzymatic dynamic kinetic resolution reaction according to Example 1 compared to a chemo-enzymatic dynamic kinetic resolution reaction according to the Reference Example.
Fig. 7 is a diagram showing the enantiomeric excess over the course of conversion for a spatially separated chemo-enzymatic dynamic kinetic resolution reaction according to Example 2.
Fig. 8 is a diagram showing the enantiomeric excess of *(S)*-2-pentanol dependent on the reaction time for a spatially separated chemo-enzymatic dynamic kinetic resolution reaction according to Example 1 compared to Example 2.

### DETAILED DESCRIPTION OF THE INVENTION

The invention relates to a process for the preparation of a product via a chemo-enzymatic reaction in a reactor comprising a first reaction zone comprising a chemo-catalyst, and second reaction zone comprising a biocatalyst, wherein the first reaction zone is operated at a first temperature T₁ to provide a first reaction mixture and the second reaction zone is operated at a second temperature T₂ which is lower than the first temperature T₁ to provide a second reaction mixture, wherein at least a part of the first reaction mixture is transferred to the second reactor zone and/or at least a part of the second reaction mixture is transferred to the first reactor zone, and wherein the product is isolated from the first reaction mixture and /or the second reaction mixture.

In a preferred embodiment, the above process according to the invention comprises
a) contacting one or more feed materials, optionally in the presence of a solvent, with the chemo-catalyst at the first temperature T₁ in the first reaction zone of the reactor to provide the first reaction mixture,
b) transferring at least a part of the first reaction mixture from the first reaction zone into the second reaction zone of the reactor,
c) contacting the at least part of the first reaction mixture with a biocatalyst at the second temperature T₂ in the second reaction zone, wherein the temperature T₂ is lower than the temperature T₁, to provide the second reaction mixture,
d) optionally recycling at least a part of the second reaction mixture from the second reaction zone into the first reaction zone,
e) optionally contacting at least a part of the second reaction mixture with the chemo-catalyst at the first temperature T₁ in the first reaction zone of the reactor to provide further first reaction mixture,
f) optionally repeating steps b) to e) one or several times,
g) isolating the product from the first and/or the second reaction mixture.

As used herein, the term "chemo-enzymatic reaction" refers to a reaction, preferably a two-step or multi-step reaction, which involves use of a chemo-catalyst and a biocatalyst. As used herein, a biocatalyst is a catalyst comprising at least one enzyme. Such chemo-enzymatic reactions are widely known in the state of the art. Preferred examples of chemo-enzymatic reactions which can be performed by the process according to the invention include chemo-enzymatic reactions selected from the group consisting of dynamic kinetic resolution, alkene epoxidation and asymmetric carbonyl reduction. According to a particularly preferred embodiment, the chemo-enzymatic reaction is a dynamic kinetic resolution.

The process according to the invention is performed in a reactor. As used herein, the term "reactor" refers to a container like a vessel including equipment which is designed to contain one or more reaction mixtures and to allow performance and control of one or more chemical reactions.

In particular, the reactor used in the process according to the invention is a one-pot reactor, i.e. a reactor capable for carrying out two or more reaction steps in one reactor without any cumbersome intermediate steps of separation, recovery and purification. Such one-pot syntheses are known to improve the efficiency of chemical two-step or multi-step reactions.

The reactor used in the process according to the invention comprises a first reaction zone and a second reaction zone. As used herein, the term "reaction zone" refers to a part or section of the reactor, which can be operated at conditions, such as temperatures, different to other parts or sections of the reactor.

According to the invention, the first reaction zone of the reactor comprises a chemo-catalyst and the second reaction zone of the reactor comprises a biocatalyst. The first reaction zone is spatially separated from the second reaction zone. Thus, the chemo-catalyst can be operated at reaction conditions which can be different from the reaction conditions applied to the biocatalyst. Furthermore, in a particularly preferred embodiment, the chemo-catalyst does not contact the biocatalyst to avoid negative effects of interaction between both catalysts. In a preferred embodiment, the first reaction zone is located at a bottom end of the reactor, particularly at the bottom end of the reactor, while the second reaction zone is located towards a top end of the reactor. Such an arrangement or reactor setup is particularly preferred with respect to the transfer of the first and second reaction mixtures, as will be described in detail below.

The first reaction zone comprising the chemo-catalyst is operated at a first temperature T₁ to provide a first reaction mixture, and the second reaction zone comprising the biocatalyst is operated at a second temperature T₂ to provide a second reaction mixture. In order to allow the chemo-enzymatic reaction to take place, at least a part of the first reaction mixture is transferred from the first reaction zone to the second reaction zone. In addition or alternatively, at least a part of the second reaction mixture is transferred from the second reaction zone to the first reaction zone. Thus, the products obtained by the chemo-catalyzed reaction in the first reaction zone are made available to the biocatalyzed reaction in the second reaction zone and/or vice versa.

Such transfer can be achieved by any transfer that allows feed materials, such as substrates, reaction products and/or solvents to pass from one reaction zone to the other reaction zone, but at the same time prevents transfer from the chemo-catalyst to the second reaction zone and transfer from the biocatalyst to the first reaction zone.

In a particularly preferred embodiment of the process according to the invention, the transfer of at least part of the first reaction mixture from the first reaction zone to the second reaction zone is performed by evaporation and optionally subsequent condensation. Preferably, the first reaction mixture is operated at conditions which allow evaporation of e.g. feed materials, reaction products and/or solvents contained in the first reaction mixture. Preferably, the feed materials are evaporated to form a vapor substantially free of solvent to allow accelerated reaction performance in the second reaction zone. The resulting vapour can then be directly passed into the second reaction zone or can be passed into a condenser to obtain a liquid fraction from the first reaction mixture which is then passed into the second reaction zone. Evaporation of the chemo-catalyst is prevented by applying evaporation conditions to the first reaction zone which are not suitable to evaporate the chemo-catalyst. This can be easily achieved taking into account that chemo-catalysts used in chemo-enzymatic reactions typically are metal catalysts that are not prone to evaporation at all. In any case, the evaporation conditions applied to the first reaction mixture are suitable to transfer those species from the first reaction mixture to the second reactor zone which are required for the biocatalyzed reaction in the second reaction zone.

Accordingly, evaporation of a part of the first reaction mixture is an easy and effective way to transfer one or more substrates, i.e. species required for the biocatalyzed reaction, from the first reaction zone to the second reaction zone without transferring the chemo-catalyst. In such an embodiment, the chemo-catalyst does not need to be supported or otherwise immobilized which is a further advantage of such a mode of transfer. This means any homogenously dissolvable chemo-catalyst which cannot be evaporated under conditions applied to the first reaction zone may be used in the first reaction zone, which can for example be a bottom of distillation.

As described above, the first reaction zone is preferably located at a bottom end of the reactor, whereas the second reaction zone is preferably located higher as the first reaction zone, i.e. towards a top end of the reactor. This reactor setup is particularly preferred in case the transfer of the at least part of the first reaction mixture to the second reaction zone is performed by evaporation in view of the fact that the obtained vapour fraction from the first reaction zone readily rises towards the top of the reactor. Moreover, in such a reactor setup the transfer of the at least part of the second reaction mixture from the second reaction zone to the first reaction zone can easily be achieved passing a liquid fraction of the second reaction mixture downwards to the first reaction zone at the bottom end of the reactor by gravitational force. A transfer of the biocatalyst from the second reaction zone to the first reaction zone can be prevented by using a supported, immobilized or otherwise fixed biocatalyst.

According to an alternative embodiment, the transfer of the first reaction mixture from the first reaction zone to the second reaction zone and/or the transfer of the second reaction mixture from the second reaction zone to the first reaction zone is performed by pumping the respective reaction mixture into the desired reaction zone. Optionally, a filter is provided within the pump line connecting the first and second reaction zone. Thus, a transfer of solids like catalyst particles, such as a supported chemo-catalyst or immobilized biocatalyst, can be prevented.

In a preferred embodiment the process according to the invention is carried out in a one-pot reactor setup as described in more detail in Example 1 below. According to this embodiment, the chemo-catalyst, one or more feed materials, which are typically the substrate materials required for the biocatalyzed reaction, and optionally one or more solvents are placed in the first reaction zone, such as a vessel at the bottom of the reactor. The first reaction zone can be heated by a heating source and, therefore, temperature T₁ can be applied to the first reaction zone providing the first reaction mixture and causing evaporation of at least a part of the first reaction mixture and provision of a vapour stream comprising substrate material and optionally solvent. The vapour stream is passed upstream to the top end of the first reaction zone into the second reaction zone. According to one embodiment, the vapour is first passed into a condenser, for example via a vapour line of a glass tube, and the liquid obtained in the condenser is passed into the second reaction zone, such as a liquid line of a vertical glass tube arranged on the top of the first reaction zone, such as a dropping funnel. Optionally, a filler, like a plurality of glass beads, is present in the second reaction zone. This facilitates arrangement of an immobilized biocatalyst in the second reaction zone. The immobilized biocatalyst can be placed either in the upper part or in the lower part of the liquid line to adjust the temperature of the immobilized biocatalyst.

Accordingly, it is particularly preferred that the first reaction zone is formed by a vessel, such as a round bottom flask, and the second reaction zone is formed by a tube, such as a glass tube like a dropping funnel which can additionally be equipped with a pressure equalizing tube.

Furthermore, it is particularly preferred that at least a part of the second reaction mixture from the second reaction zone is recycled into the first reaction zone. Consequently, it is mixed with the first reaction mixture. Thereby, unreacted substrate materials from the second reaction mixture are repeatedly contacted with the chemo-catalyst in the first reaction zone. Contacting of unreacted substrate materials with the chemo-catalyst, for example, causes racemization of the slower reacting enantiomer in dynamic kinetic resolution and, therefore, new fast reacting enantiomer is obtained which gives an increased total conversion of the racemic substrate material of more than 50%. Hence, the process according to the invention can be a recirculation procedure.

It is preferred that the obtained one or more products are isolated by processes which are generally used in syntheses. Such processes are, for example, extraction, fractional distillation or precipitation.

The temperature T₁ of the first reaction zone comprising the chemo-catalyst is higher than the temperature T₂ of the second reaction zone comprising the biocatalyst. Preferred temperature values T₁ and T₂ may vary depending on the nature of the chemo-enzymatic reaction, the catalysts employed and the nature of the reactants and products and optionally solvents. However, it is generally preferred that the first temperature T₁ is in the range of 30 to 250 °C, preferably 50 to 200 °C, more preferably 70 to 190 °C, such as 70 to 150 °C. The first temperature T₁ of the first reaction zone should be suitable to operate a chemo-catalyzed reaction.

Moreover, it is generally preferred that the second temperature T₂ is in range of 120 to 10 °C, preferably 80 to 20 °C, more preferably 40 to 25 °C. The second temperature T₂ of the second reaction zone should be suitable to operate an enzymatic reaction. In particular, deactivation of the biocatalyst should be avoided or at least reduced.

The given temperature ranges can be modified by applying vacuum conditions to reduce boiling temperatures of the evaporated reactants.

It is further preferred that the process according to the invention exhibits a difference between temperatures T₁ and T₂ of more than 10 °C, preferably more than 50 °C and more preferably more than 100 °C. It is believed that a higher temperature difference may in many cases increase the activity of the chemo-catalyst following Arrhenius equation.

In another embodiment of the process according to the invention the pressure of the atmosphere within the reactor is reduced. Reducing the pressure lowers the boiling points of given solvents, substrates and/or products. Thus, the composition of the first and/or second reaction mixture can be modified or evaporation of components of the first reaction mixture can be facilitated.

According to a preferred embodiment, the chemo-catalyst used in the process according to the invention is selected from the group consisting of metal catalysts comprising a metal from Groups 3 to 15 of the Periodic Table of Elements, such as vanadium, ruthenium, rhodium, palladium or aluminium; an acidic or alkaline catalyst, such as acidic ion exchange resin, or homogenously dissolvable acids and bases without support on resin material, and hydrophilic or hydrophobic zeolite materials or combinations thereof.

According to another preferred embodiment, the biocatalyst used in the process according to the invention is selected from the group consisting of lipases or reductases, such as lipases from *Candida Antarctica, Candida rugosa, Pseudomonas sp., Mucor miehei* or reductase from Candida parapsilosis, or a combination thereof. More preferably, preparations of *Candida Antarctica* such as *Candida Antarctica* lipase B (CalB) are chosen, e.g. Novozym435 or c-LEcta. Compatibility of biocatalyst with organic solvents is generally desired.

As described above, it is preferred that the chemo-enzymatic reaction performed by the process according to the invention is a dynamic kinetic resolution. Such dynamic kinetic resolution reactions are particularly suitable to be performed in the one-pot reactor setup described above giving the opportunity to operate the corresponding chemo-catalyst at an optimal high temperature with simultaneously increased operation time of the biocatalyst.

In a further preferred embodiment, the dynamic kinetic resolution is a dynamic kinetic resolution of secondary alcohols, secondary amines or allyl esters. In a dynamic kinetic resolution both enantiomers of a racemic compound can be converted to an enantiopure product in an asymmetric synthesis using a catalyst combination of a chemo-catalyst and a biocatalyst. The enantiomers of a racemate are dynamically altered into each other in an equilibrium reaction. This reaction is catalyzed by the chemo-catalyst. The biocatalyst shows high preference for one of both enantiomers which is converted to the final product. The enzymatic less reactive enantiomer remains in the reaction mixture and, constantly, undergoes racemization by the chemo-catalyst regenerating enzymatic preferred enantiomer. Thus, both enantiomers are consumed and even full conversion of a racemate into one enantiopure product is obtainable.

In a particularly preferred embodiment of the process according to the invention, the chemo-enzymatic reaction is a dynamic kinetic resolution of a secondary alcohol or amine and the biocatalyst is CalB absorptively connected to a polyacrylic resin (known as Novozym435) and the chemo-catalyst is a ruthenium catalyst, such as Shvo catalyst of Formula I

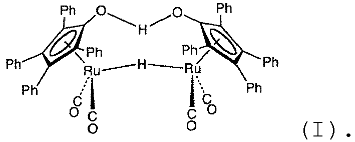

The first temperature T₁ preferably is between 70 and 200 °C, more preferably between 170 and 190 °C and the second temperature T₂ preferably is between 20 and 60 °C, more preferably between 29 and 33 °C.

The substrates of such a reaction are for example (R/S)-2-pentanol and ethyl butyrate. Additionally or alternatively, different chain lengths and configurations of the chiral alcohol species, e.g. *(R*/*S)*-3-methyl-2-butanol, *(R*/*S)*-2-methyl-3-pentanol, *(R*/*S)*-2-hexanol as well as the corresponding ester compounds e.g. methyl/propyl butyrate, methyl/ethyl/propyl isobutyrate, methyl/ethyl/propyl acrylate can be applied.

Furthermore, it is preferred that the solvent of such a reaction is selected from xylene, preferably p-xylene, or toluene. It is particularly preferred that the boiling temperature of the solvent material is higher compared to the boiling temperature of substrate species, independent from the applied operating pressure.

In a preferred embodiment of the process according to the invention a dynamic kinetic resolution using *(R*/*S)*-2-pentanol and ethyl butyrate as substrate materials solved in p-xylene is performed. Dynamic kinetic resolution to the corresponding products *(R)*-pentyl butyrate and ethanol is preferably catalyzed by the immobilized *Candida antarctica* lipase B (CalB) as biocatalyst and a metal catalyst including ruthenium as chemo-catalyst, such as Shvo catalyst of Formula (I), as illustrated in the following reaction scheme:

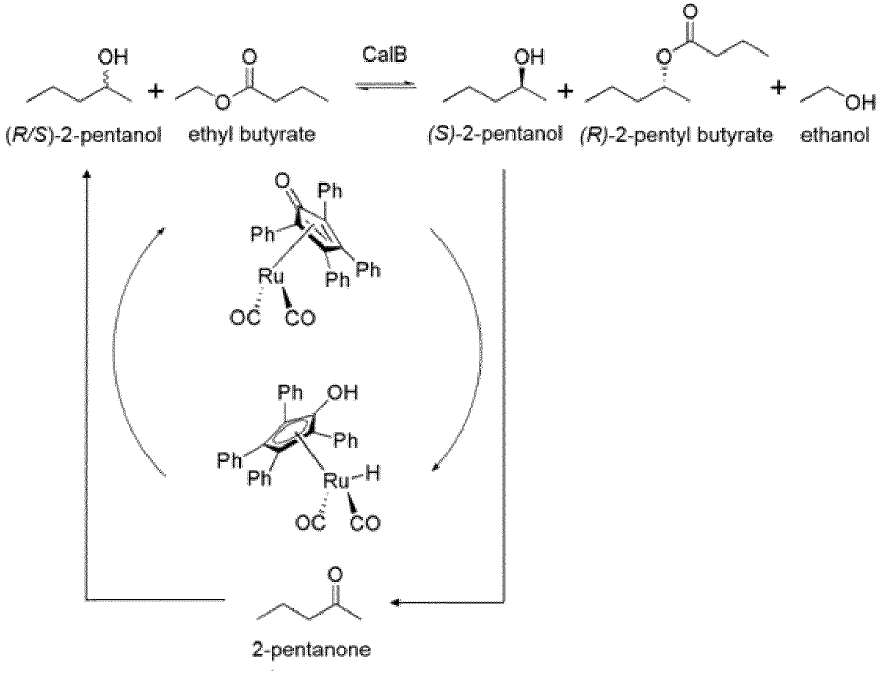

The invention will be further illustrated by way of the following examples.

### EXAMPLES

### Chemicals

*(R*/*S)*-2-pentyl butyrate (≥98%), (R/S)-2-pentanol (≥98%), ethyl butyrate (≥98%), 2-pentanone (≥98%) and p-xylene were purchased from Sigma Aldrich. Ethanol (≥99.8%), ethyl acetate (≥99.5%), sodium carbonate anhydrous (≥99.5%) and molecular sieve 4 Å (Type 514, 0.4 nm, pearl form) were purchased from Roth.

Prior to the experiments described below, pre-distillation of ethyl butyrate and p-xylene was performed. *(R*/*S)*-2-pentanol was dried on molecular sieve before use. Storage of distilled chemicals took place in a desiccator. The pre-treatment of chemicals by distillation was performed to avoid impurities of water or other oxidizing molecules.

The preparation Novozym435® (CAS: 9001-62-1) was used as biocatalyst in the following experiments. It consists of *Candida antarctica* lipase B (CalB) adsorbed and, therefore, immobilized on a macroporous, polyacrylic resin. According to the manufacturer, CalB for Novozym435® is produced by submerged fermentation of a genetically modified *Aspergillus niger* microorganism. Activity is described to be ≥ 5000 U·g⁻¹ as obtained in propyl laurate assay.

As chemo-catalyst, the commercially available Shvo catalyst of Formula (I) was used.

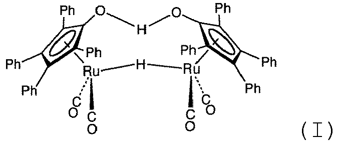

### GC-Analysis

Sample analysis was carried out at an Agilent7890B gas chromatograph (GC) equipped with a CP-Chirasil-DEX CB column (L_{column}=25 m, dᵢ=0.25 mm). Hydrogen served as carrier gas in the column and helium was applied as make-up gas for the flame ionization detector (FID). Prior to injection, sample treatment involved 1:100 (v/v) dilution in ethyl acetate. The adjusted parameters and the retention times for GC analysis are presented in Table 1.

**Table 1**

| **GC method** | | **Retention times** | |
|---|---|---|---|
| **Parameter** | **Value** | **Reactant** | **t[min]** |
| V_{injection} | 1 µL | EtOH | 1.3 |
| Flow | 1.5 mL min⁻¹ | EtOAc | 1.6 |
| Tᵢₙₗₑₜ | 250 °C | 2-pentanone | 2.8 |
| Tₛₜₐᵣₜ | 50 °C | EtBu | 5.1 |
| T_{Ramp 1} | 85 °C (10 °C·min⁻¹) | *(R)*-2-PeOH | 6.2 |
| T_{Ramp 2} | 120 °C (30 °C·min⁻¹) | *(S)*-2-PeOH | 6.4 |
| T_{detector} | 300 °C | *p*-xylene | 6.6 |
| t per sample | 10.7 min | *(S)*-2-PeBu | 9.5 |
| Mode | Split (50:1) | *(R)*-2-PeBu | 9.7 |

- EtOH: = ethanol
- EtOAc: = ethyl acetate
- EtBu: = ethyl butyrate
- PeOH: = pentanol
- PeBu: = pentyl butyrate

### Reference Example: Dynamic kinetic resolution with non-separated catalysts in one reaction zone

The amounts listed in Table 2 were placed in the bottom of an evacuated batch vessel (reactor volume = 5 mL). The obtained reaction mixture was heated to three different temperatures: 32 °C (Option A), 65 °C (Option B) and 72 °C (Option C) for 240 min (Option A), 90 min (Option B), and 90 min (Option C), respectively. As both catalysts were present at the same position in the batch vessel, no temperature difference between the catalysts was present. This corresponds to the state-of-the-art procedure for carrying out chemo-enzymatic dynamic kinetic resolution reactions.

**Table 2**

| Parameter | Option A | Option B | Option C |
|---|---|---|---|
| T [°C] | 32 | 65 | 72 |
| Shvo catalyst [g] | 0.022 | 0.022 | 0.011 |
| Ca1B [g] | 0.035 | 0.035 | 0.105 |
| Na₂CO₃ [g] | 0.133 | 0.133 | 0.068 |
| *p*-xylene [g] | 0.506 | 0.127 | 0.282 |
| EtBu [g] | 1.078 | 1.089 | 0.562 |
| *(R*/*S)*-2-PeOH [g] | 0.430 | 0.150 | 0.147 |

| | | | |
|---|---|---|---|
| EtBu = ethyl butyrate PeOH = pentanol | | | |

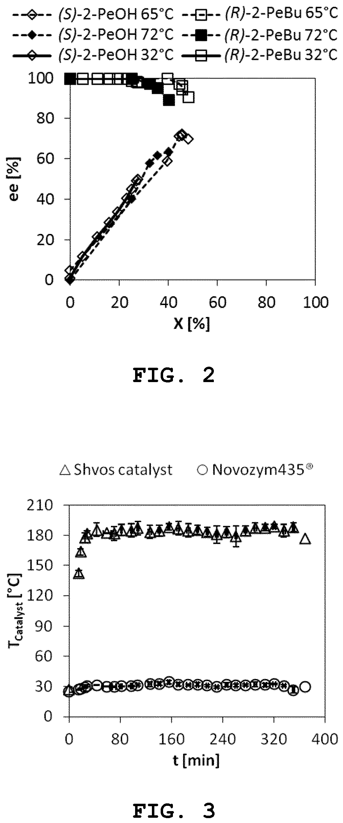

As can be seen from Figure 2 and Table 3, an enantiomeric excess of *(R)*-2-PeBu of more than 89% was observed at all three reaction temperatures up to a conversion of (R/S)-2-PeOH close to 50% (Option B). This shows that the stereoselective transesterification catalyzed by the biocatalyst took place at all three reaction temperatures.

**Table 3**

| Parameter | Option A | Option B | Option C |
|---|---|---|---|
| T [°C] | 32 | 65 | 72 |
| ee_{*(R)*-2-PeBu} [%] | 97.9 | 90.9 | 89.3 |
| ee_{*(S)*-2-PeOH} [%] | 49.9 | 69.9 | 63.6 |
| X_{*(R*/*S)*-2-PeOH} [%] | 27.7 | 48.1 | 40.1 |

Moreover, a comparable increase in enantiomeric excess of *(S)-*2-PeOH was observed for all three reaction temperatures. This shows that the chemo-catalyzed racemization did not take place at a satisfactory rate. Apparently, the chemo-catalyst exhibited no substantial catalytic activity at the investigated temperature range from 32 to 72 °C.

### Example 1: Dynamic kinetic resolution of (R/S)-2-pentanol with ethyl butyrate and separated catalysts

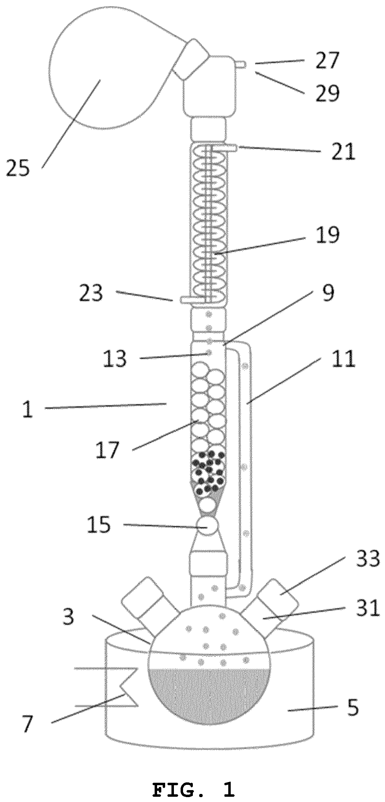

A one-pot reactor setup according to Figure 1 was used. The reactor setup (1) had a total height of 600 mm and comprised a 50 mL glass vessel (3) in a heating bath (5) with a heating source (7). Vessel (3) was suitable to receive Shvo catalyst of Formula (I), feed materials like the substrates *(R*/*S)*-2-pentanol and ethyl butyrate as well as solvent such as p-xylene forming the first reaction mixture. Thus, upon operation it was possible that the first reaction mixture including the chemo-catalyst was exposed to rather high temperatures such as the boiling temperature of the first reaction mixture. On top of the reaction vessel (3) a glass tube (9) (h = 120 mm) with two separate lines, namely a vapor line (11) and a liquid line (13) was placed. The vapor line (11) had a height of 150 mm. Its bottom end was connected to the top end of vessel (3) to allow vapor from vessel (3) to rise to the top of tube (9). The liquid line (13) had a height of 120 mm. Its bottom end was connected to the top end of vessel (3) via a valve (15) which allows liquid to pass from the liquid line (13) down into vessel (3). The liquid line (13) was filled with glass beads having a diameter of 2 to 3 mm as a filler (17). The filler (17) was placed in the liquid line such that the immobilized biocatalyst can be placed within the glass beads (17), for example either in the upper part (such as h = 80-100 mm) of the liquid line (13) or in the lower part (such as h = 20-40 mm) of the liquid line (13). Thus, the temperature at which the biocatalyst is operated can be influenced by the position of the catalyst in the liquid line (13). At the top end of glass tube (9), the top end of vapor line (11) was connected to the top end of liquid line (13) allowing fluid communication between the top end of vapor line (11) and liquid line (13). The top end of the glass tube (9) was in fluid communication with a reflux condenser (19) having an inlet (21) and an outlet (23) for cooling water. Uprising vapor from vessel (3) was allowed to pass the vapor line (11) and to arrive at condenser (19). Due to the low temperature of the cooling water in condenser (19), the vapor condensed in the condenser (19) to obtain a liquid which was allowed to flow downstream from the condenser (19) into the liquid line (13) through the bed of filler (17) thereby contacting the immobilized biocatalyst. The valve (15) was slightly open during operation to allow liquid flow back into the vessel (3) at the bottom of the reactor setup (1). A nitrogen balloon (25) was connected to the top of reflux condenser (21) to prevent overpressure and simultaneously avoid oxygen from air flushing into the system. At the top of reflux condenser (21) an inlet (27) for nitrogen flow and an inlet (29) for applying vacuum were provided. To be flexible in changing between nitrogen flow and vacuum, inlets (27) and (29) can be connected to a Schlenk line system (not shown in Figure 1). The vessel (3) also had an opening (31) which is equipped with a septum (33) which allows to take samples of the first reaction mixture contained in vessel (3) or to measure the temperature of the reaction mixture contained in vessel (3).

Glassware was flamed under clean bench prior to use to get rid of moisture. Two times switching between nitrogen filling and vacuum conditions in the system was performed to ensure absence of molecular oxygen. All connection parts were sealed with Teflon tape and clips to prevent oxygen species in the system during operation. Chemicals were added under nitrogen flow at room temperature.

0.25 g of immobilized CalB were placed in the lower part of the liquid line (h = 20-40 mm). Then, 0.16 g of Shvo catalyst and 1 g of Na₂CO₃ were added to the vessel (3) at the bottom of the setup (1). Subsequently, 3 g of p-xylene (0.23 mol%), 2.45 g of *(R*/*S)*-2-PeOH (0.23 mol%), 7.51 g of EtBu (0.54 mol%) were added to generate the initial reaction mixture at the bottom of the setup (1). In order to prevent oxygen species in the setup, addition of chemicals is performed under presence of nitrogen atmosphere in the storage flasks of *p*-xylene, *(R*/*S)*-2-PeOH and EtBu. The withdrawal out of the storage flasks and the addition of *p*-xylene, *(R*/*S)*-2-PeOH and EtBu was realized by syringes via septa. The weight ratio of CalB to Shvo catalyst was fixed to 1.56. The additional sodium carbonate was placed in the reactor to support the absence of oxidizing species by drying the solvent mixture. Afterwards evacuation was performed again by flushing nitrogen and sucking vacuum three times. No nitrogen flowed through the reactor setup during the experiment, because low boiling compounds could falsify mass balance and concentration profiles. To prevent delay in boiling in the vessel at the bottom of the setup, mixing was performed with a magnetic stirrer. The employed operation conditions are shown in Table 4.

**Table 4**

| **Parameter** | **Value** |
|---|---|
| p [mbar] | 1013 |
| X_{EtBu, 0} [mol·mol⁻¹] | 0.54 |
| *x*_{*(R*/*S)*-2-PeOH, 0} [mol·mol⁻¹] | 0.23 |
| x_{*p*-xylene, 0} [mol·mol⁻¹] | 0.23 |
| M_{Novozym435®} [g] | 0.25 |
| m_{Shvo catalyst} [g] | 0.16 |
| m_{Na2CO3} [g] | 1 |
| m₀ [g] | 13 ± 0.1 |
| T_{Shvo,mean} [°C] | 183 ± 5 |
| T_{Novozym435®,mean} [°C] | 31 ± 2 |

| | |
|---|---|
| m₀ = initial mass of starting materials (p-xylene, *(R*/*S)*-2-PeOH and EtBu) | |

Over the course of the reaction, which took 369 min, the temperature was monitored at the bottom of the setup (1) in vessel (3) (T_{Shvo}) and at the position of the biocatalyst in the liquid line (13) (T_{Novozym435®}). The temperature in the bottom of the setup (1) (T_{Shvo}) was measured in the oil bath, which was applied for heating vessel (3). Temperature measurement in the liquid line (T_{Novozym435®}) was performed externally via an infrared thermometer. The temperature at Shvo catalyst (T_{Shvo}) was 183 ± 5 °C and the temperature at the position of the biocatalyst (T_{Novozym435®}) was 31 ± 2 °C. Hence, the resulting temperature difference was 152 ± 4 °C. The temperatures observed during Example 1 are shown in Figure 3.

Accordingly, increased temperatures at the bottom accelerated the reaction rate of the chemo-catalyst based on Arrhenius dependency. At the same time, lower temperatures were present at the position of the biocatalyst to reduce thermal deactivation. This allowed operation of both catalysts at preferred conditions and limitations with respect to a common operating temperature for two different catalysts were prevented.

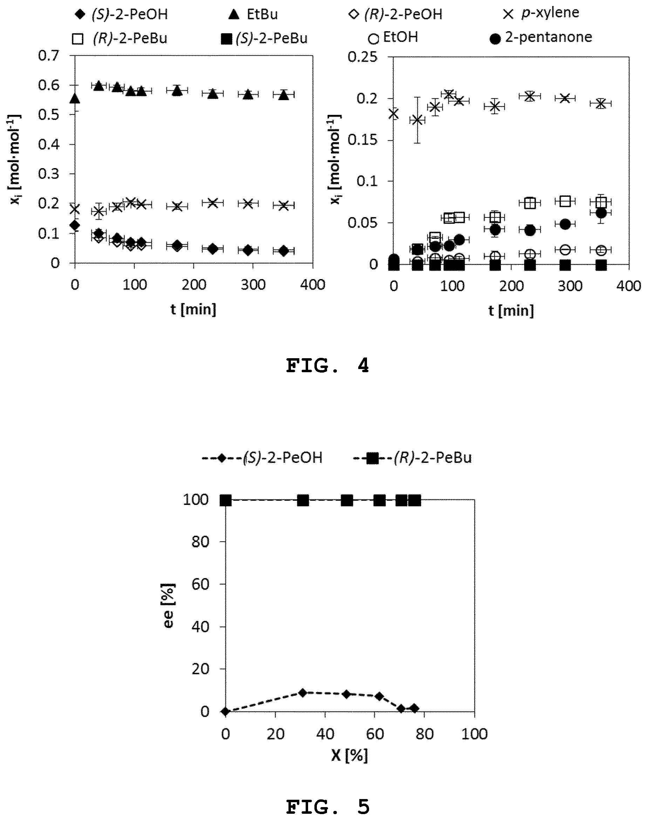

The obtained course of reaction is shown in Figure 4 for all reactants. In the left plot, the molar ratios of the substrates *(S)*-2-PeOH, (R)-2-PeOH and EtBu and the solvent p-xylene are shown in dependency of reaction time. A decrease of both enantiomers of 2-PeOH was observed with a slightly faster decrease of *(R)*-2-PeOH, which - without wishing to be bound to a particular theory - can be explained by the *(R)*-selectivity of the enzymatic reaction in the liquid line of the setup. Subsequent racemization of the residual mixture of *(R)*-2-PeOH and *(S)*-2-PeOH catalyzed by Shvo catalyst in the bottom led to formation of racemic *(R*/*S)*-2-PeOH. The molar ratio of EtBu shows an initial increase and a slight decrease afterwards, whereas the molar ratio of *p*-xylene is slightly increased over the course of reaction. This behavior can be explained by faster evaporation of lower boiling *(R*/*S)*-2-PeOH and EtBu compared to the solvent *p*-xylene.
In the right plot, product concentrations as well as the concentration of solvent material are demonstrated. Small molar ratios of ethanol were observed, which was mainly present in the vapor phase due to its low boiling point of 78 °C at ambient pressure. Enantioselective formation of *(R)-*2-PeBu shows that the biocatalytic reaction proceeded successfully. Only small differences in the molar ratio of both enantiomers of 2-PeOH were obtained. This shows that racemization and enzymatic reaction took place at the same time at their different positions. Moreover, it can be seen from Fig. 3 that side product formation of 2-pentanone took place in the racemization reaction in similar molar ratios compared to the target product *(R)*-2-PeBu. The error determination is given as standard deviation between two separate experiments with an overall calculated mass balance error of 5.7 ± 0.9 %.

Selectivity of the enzymatic reaction was investigated by comparing enantiomeric excess over the course of conversion (see Fig. 5). The enantiomeric excess of the target product *(R)*-2-PeBu was higher than 99% up to conversions of 76% (see Table 5, With T-difference). Hence, high enantioselectivity of the biocatalyst preparation Novozym435^{®} in terms of selective formation of *(R)*-2-PeBu is demonstrated. At the same time, the enantiomeric excess of *(S)*-2-PeOH stayed lower than 9% due to effective racemization in the chemo-catalyzed reaction step of the dynamic kinetic resolution reaction (see Table 5, With T-difference). Interference of the catalysts was prevented by spatial separation.

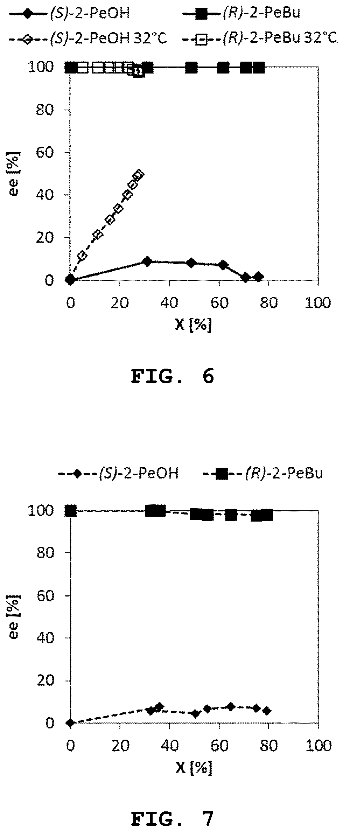

The previously discussed results obtained with a temperature difference between both reaction zones are in the following compared with the Reference Example to evaluate applicability of a chemo-enzymatic dynamic kinetic resolution with spatial separation of the catalysts. Figure 6 demonstrates advantages obtained with the spatial separation of the catalyst as can be seen from the constantly low ee-values for *(S)*-2-PeOH (<9%) in case a temperature difference is applied. Without a temperature difference and operating both catalysts at 32 °C, which is the mean temperature at the position of the biocatalyst in Example 1, expected behavior of rising ee-values for residual *(S)*-2-PeOH is observed, which shows that the racemization reaction is insufficiently catalyzed (Table 5, Without T-difference).

**Table 5**

| With T-difference | | | Without T-difference | | |
|---|---|---|---|---|---|
| X _{*(R*/*S)-*2-PeOH} [%] | ee _{*(R)*-2-PeBu} [%] | ee _{*(S)*-2-PeOH} [%] | X _{*(R*/*S)*-2-PeOH} [%] | ee _{*(R)*-2-PeBu} [%] | ee _{*(S)*-2-PeOH} [%] |
| 0 | > 99 | 0 | 0 | > 99 | 0.7 |
| 31.0 | > 99 | 8.9 | 5.0 | > 99 | 11.6 |
| 48.7 | > 99 | 8.2 | 11.2 | > 99 | 21.6 |
| 61.6 | > 99 | 7.2 | 16.1 | > 99 | 28.5 |
| 70.7 | > 99 | 1.3 | 19.4 | > 99 | 33.8 |
| 75.9 | > 99 | 1.6 | 23.1 | > 99 | 40.4 |
| 75.8 | > 99 | 1.7 | 25.2 | 98.9 | 44.9 |
| | | | 27.2 | 98.5 | 49.0 |
| | | | 27.7 | 97.9 | 49.9 |

### Example 2: Dynamic kinetic resolution of (R/S)-2-pentanol with ethyl butyrate and separated catalysts

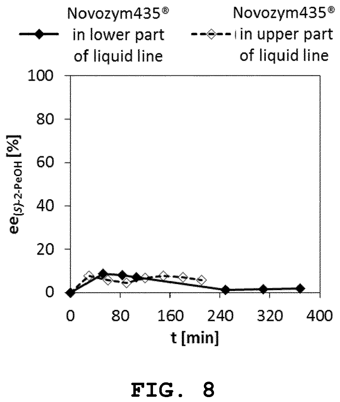

The experiment of Example 1 was repeated except that the biocatalyst was placed in the upper part of the liquid line, which resulted in an increased operating temperature at the position of the biocatalyst (T_{Novozym435®} = 64 ± 12 °C) and a reduced temperature difference between the spatially separated catalysts of 133 ± 8 °C. The temperature at the position of the racemization catalyst (T_{Shvo}) was 196 ± 11°C. The enantiomeric excess over the course of conversion (Figure 7) was comparable to the results obtained with a higher temperature difference in Example 1. The enantiomeric excess of *(R)*-2-PeBu was high (> 97.9 %) whereas the enantiomeric excess of *(S)*-2-PeOH was constantly low (< 8 %). This behavior documented successful catalysis of both reactions up to conversions of 79 %. Due to increased temperatures at the position of the biocatalyst, a faster reaction took place (Figure 8).

## Claims

1. A process for the preparation of a product via a chemo-enzymatic reaction in a reactor comprising a first reaction zone comprising a chemo-catalyst, and second reaction zone comprising a biocatalyst,
wherein the first reaction zone is operated at a first temperature T₁ to provide a first reaction mixture and the second reaction zone is operated at a second temperature T₂ which is lower than the first temperature T₁ to provide a second reaction mixture, wherein at least a part of the first reaction mixture is transferred to the second reactor zone and/or at least a part of the second reaction mixture is transferred to the first reactor zone, and
wherein the product is isolated from the first reaction mixture and /or the second reaction mixture.

2. The process according to claim 1 comprising
a) contacting one or more feed materials, optionally in the presence of a solvent, with the chemo-catalyst at the first temperature T₁ in the first reaction zone of the reactor to provide the first reaction mixture,
b) transferring at least a part of the first reaction mixture from the first reaction zone into the second reaction zone of the reactor,
c) contacting the at least part of the first reaction mixture with a biocatalyst at the second temperature T₂ in the second reaction zone, wherein the temperature T₂ is lower than the temperature T₁, to provide the second reaction mixture,
d) optionally recycling at least a part of the second reaction mixture from the second reaction zone into the first reaction zone,
e) optionally contacting at least a part of the second reaction mixture with the chemo-catalyst at the first temperature T₁ in the first reaction zone of the reactor to provide further first reaction mixture,
f) optionally repeating steps b) to e) one or several times,
g) isolating the product from the first and/or the second reaction mixture.

3. The process according to claim 1 or 2, wherein at least part of the first reaction mixture is transferred from the first reaction zone to the second reaction zone by evaporation and optionally condensation.

4. The process according to any of claims 1 to 3, wherein the first reaction zone is a vessel, and the second reaction zone is a liquid line.

5. The process according to any of claims 1 to 4, wherein the first temperature T₁ is in range of 30 to 250 °C, preferably 50 to 200 °C, more preferably 70 to 190 °C and most preferably 80 to 150 °C.

6. The process according to any of claims 1 to 5, wherein the second temperature T₂ is in range of 120 to 10 °C, preferably 80 to 20 °C, more preferably 40 to 25 °C.

7. The process according to any of claims 1 to 6, wherein the difference between temperatures T₁ and T₂ is more than 10 °C, preferably more than 50 °C and more preferably more than 100 °C.

8. The process according to any of claims 1 to 7, wherein the chemo-enzymatic reaction is selected from the group consisting of dynamic kinetic resolution, alkene epoxidation and asymmetric carbonyl reduction.

9. The process according to any of claims 1 to 8, wherein the chemo-catalyst is selected from the group consisting of metal catalysts comprising a metal from Groups 3 to 15 of the Periodic Table of Elements, such as vanadium, ruthenium, rhodium, palladium or aluminium; an acidic or alkaline catalyst, such as acidic ion exchange resin or homogenously dissolvable acids and bases without support on resin, and hydrophilic or hydrophobic zeolite materials or combinations thereof.

10. The process according to any of claims 1 to 9, wherein the biocatalyst is selected from the group consisting of lipases or reductases, such as lipases from *Candida Antarctica, Candida rugosa, Pseudomonas sp., Mucor miehei* or reductase from Candida parapsilosis or a combination or a preparation, such as *Candida Antarctica* lipase B (CalB), thereof.

11. The process according to any of claims 1 to 10, wherein the chemo-enzymatic reaction is a dynamic kinetic resolution of secondary alcohols, secondary amines or allyl esters.

12. A process according to claim 11, wherein the chemo-enzymatic reaction is a dynamic kinetic resolution of a secondary alcohol or amine, the biocatalyst is CALB and the chemo-catalyst is a Shvo catalyst of Formula I

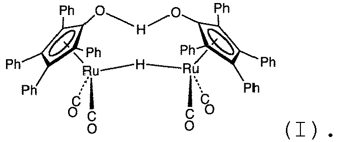

13. The process according to any of claims 11 or 12, wherein the first temperature T₁ is between 178 and 188 °C and the second temperature T₂ is between 29 and 33 °C.

14. The process according to any of claims 11 to 13, wherein the substrates are (R/S)-2-pentanol and ethyl butyrate.

15. The process according to any of claims 11 to 14, wherein the solvent is xylene, preferably p-xylene.
